# EUROPÄISCHE PATENTSCHRIFT

(11) **EP 4 121 157 B1**
(45) Veröffentlichungstag und Bekanntmachung des Hinweises auf die Patenterteilung: **01.05.2024**
(21) Anmeldenummer: 21714627.3
(22) Anmeldetag: 18.03.2021
(51) Int. Cl.: A61M 25/06, A61M 25/09, A61M 5/32

(54) **VERWEILKANÜLE**
INDWELLING CANNULA
CANULE À DEMEURE

(30) Priorität: 20.03.2020 DE 102020107773
(43) Veröffentlichungstag der Anmeldung: 25.01.2023
(73) Patentinhaber: BUTZ, Martin, 66497 Contwig (DE)
(72) Erfinder: BUTZ, Martin, 66497 Contwig (DE)
(74) Vertreter: Patentanwaltskanzlei Vièl & Wieske PartGmbB
(86) Internationale Anmeldenummer: PCT/DE2021/100274
(87) Internationale Veröffentlichungsnummer: WO 2021/185417

(56) Entgegenhaltungen:
- EP-A1- 3 131 466
- JP-A- H10 179 734
- US-A- 4 944 728
- US-A1- 2013 178 711
- US-A1- 2017 239 443
- US-A1- 2019 351 196

## Beschreibung

Die Erfindung betrifft eine Verweilkanüle mit einem flexiblen Katheter und einer Hohlnadel, die mittels einer Überwurfmutter mit einem Gehäuse verbindbar ist, wobei der Katheter innerhalb der Hohlnadel längsverschiebbar ist und frei durch das Gehäuse verläuft. Insbesondere betrifft die Erfindung eine Verweilkanüle mit Rückschubschutz der Punktionsnadel.

Dabei ist die distale Seite der Verweilkanüle die vom Patienten abgewandte Seite der Verweilkanüle und die proximale Seite der Verweilkanüle die dem Patienten zugewandte Seite der Verweilkanüle.

Verweilkanülen werden zur Punktion von Körperhöhlen, beispielsweise bei Pleurapunktionen, oder von Gefäßen, beispielsweise von Venen oder Arterien, eingesetzt. Im Folgenden wird die Erfindung am Beispiel einer Venenverweilkanüle beschrieben.

Die Zufuhr von Flüssigkeiten in das Blutsystem und gegebenenfalls die Entnahme von Blut aus dem Blutsystem des Patienten erfolgen im Allgemeinen über einen venösen Gefäßzugang, in bestimmten Fällen auch über arterielle Gefäßzugänge. Dieser Venenzugang kann durch eine Kanüle bzw. einen Katheter erfolgen. Für den Venenzugang muss daher die Vene punktiert werden.

Zu diesem Zweck sind Venenverweilkanülen, auch Venenverweilkatheter genannt, bekannt. Diese bestehen in der Regel aus zwei Teilen, einem flexiblen Katheter einschließlich einer Fixier- und Anschlusseinheit und einer in den Katheter eingeführten Hohlnadel mit geschliffener Spitze. Dabei wird das proximale Ende des flexiblen Katheters in die punktierte Vene eingeführt. Nach dem Einführen verbleibt der flexible Katheter im Patienten und dient der Flüssigkeitszufuhr bzw. Flüssigkeitsentnahme über die Anschlusseinheit. Die Anschlusseinheit kann dabei ein Luer-Lock-Anschluss sein. Die Fixiereinheit hält die Venenverweilkanüle dabei in ihrer eingesetzten Position. Die Hohlnadel dient zum Punktieren der Vene, sowie zum Stabilisieren des flexiblen Katheters beim Einführen. Die in den Katheter eingesetzte Hohlnadel besitzt eine Schneidekante, die aus dem proximalen Ende des flexiblen Katheters herausragt. Beim Einsetzen der Venenverweilkanüle werden mit Hilfe der Schneidekante die Haut und die zu punktierende Vene punktiert. Die erfolgreiche Punktion der Vene kann dabei über den Blutrückfluss am anderen Ende der Hohlnadel erkannt werden. Anschließend wird die Hohlnadel mit dem sie umschließenden flexiblen Katheter so weit in die Vene eingeschoben, bis der flexible Katheter mit seinem proximalen Ende in die Vene eindringt. Danach wird die Hohlnadel aus dem flexiblen Katheter zurückgezogen, so dass nur der flexible Katheter im Körper des Patienten verbleibt. Dabei gilt bei der ganzen Prozedur, dass eine zurückgezogene Punktionsnadel niemals wieder im oder in den Katheter vorgeschoben werden darf.

Von der zurückgezogenen Hohlnadel geht dabei aufgrund ihres Kontaktes mit dem Blut des Patienten eine erhöhte Infektionsgefahr, insbesondere für das behandelnde medizinische Personal, aus. In den Technischen Regeln für Biologische Arbeitsstoffe (TRBA 250) werden beispielsweise Maßnahmen zur Vermeidung von Stichverletzungen empfohlen. Zugleich sollte sichergestellt werden, dass die Hohlnadel nicht wiederverwendet wird.

Aus diesem Grund sind mehrere Vorrichtungen zum Spitzenschutz von gebrauchten Hohlnadeln bekannt.

Die DE 299 21 084 U1 betrifft einen Kurzkatheter, bei dem die in das Katheterrohr eingeschobene Nadel nach dem Herausziehen durch einen Nadelschutz unbrauchbar gemacht wird. Der Kurzkatheter besteht aus ein Katheterrohr, das am Ende einen Katheteransatz aufweist und einer durch das Katheterrohr steckbaren Nadel, die am proximalen Ende einen Nadelansatz aufweist, wobei der Nadelansatz einen Vorsprung hat, welcher in den Innenraum des Katheteransatzes hineinragt. In einen Innenraum des Katheteransatzes ist ein auf der Nadel angeordneter Nadelschutz aus einem Federelement, das Löcher für den Durchgang der Nadel und am distalen Ende ein Hakenteil aufweist, eingebracht. Dabei ist der Nadelschutz in dem Katheteransatz in axial gespanntem Zustand enthalten, wobei seine Enden gegen axiales Ausdehnen blockiert sind. Der Nadelschutz hat hierbei in seiner die Nadelspitze überdeckenden Position infolge von Klemmwirkung einen festen Halt. Die Nadel kann somit eine zylindrische Rundnadel ohne jegliche Einkerbungen, Vorsprünge oder Unrundheiten sein.

Die DE 690 16 351 T2 beschreibt eine Sicherheitskappe für eine gebrauchte medizinische Nadel. Diese Sicherheitskappe erlaubt das aktive Einführen einer kontaminierten Nadel.

Die DE 20 2009 011 253 U1 betrifft eine medizinische Kanüle mit einer an einem Halter fixierten Nadel und einer Schutzhülse für die Nadelspitze. Die Schutzhülse ist dabei durch ein nur auf eine begrenzte Länge ausziehbares flexibles Zugglied mit dem Halter verbunden. Das Zugglied kann dabei ein von der Nadel durchsetztes Zugglied oder ein Kabel aus Kunststoff sein, so dass sie in einem Zustand vor Gebrauch der Kanüle in geringem Abstand zu dem Halter auf der Nadel angeordnet werden kann. Wird die Nadel herausgezogen, so wird das Zugglied auf seine maximale Länge ausgezogen, so dass es die Schutzhülse in einer Position hält, in der sie die Nadelspitze schützt.

Die DE 20 2011 052 035 U1 beschreibt eine Nadel zum Einstechen durch die Haut eines Patienten, mit einer auf der Nadel verschiebbaren Schutzhülse, die in einer Position fixierbar ist, in der sie die Nadelspitze abdeckt. Dabei weist die Schutzhülse einen rohrförmigen Fortsatz auf, der die Nadel umgibt und dazu ausgebildet ist, zusammen mit der Nadel die Haut des Patienten zu durchstechen. Wird die Nadel nach dem Durchstechen der Haut zurückgezogen, sorgt die größere Reibung zwischen dem rohrförmigen Fortsatz und dem Gewebe des Patienten dafür, dass die Nadel allein zurückgezogen wird und sich die Schutzhülse zum vorderen Ende der Nadel verschiebt. Erst dann wird die Schutzhülse von der Nadel mitgenommen, so dass auch der Fortsatz wieder aus dem Gewebe herausgezogen wird. Die Schutzhülse ist dann in einer Position an der Nadelspitze fixiert, in der sie verhindert, dass sich jemand an der Spitze der Nadel verletzt.

Die US 2004/0143195 A1 beschreibt eine abschirmbare Nadelvorrichtung. Die abschirmbare Nadelvorrichtung beinhaltet einen Ansatz mit einem Hebel, der sich von einer Außenfläche desselben erstreckt, und einer Nadelkanüle mit einer sich von dem Ansatz aus erstreckenden Einstechspitze. Das Gehäuse der Nadelvorrichtung beinhaltet einen Spitzenschutz, der die Nadel beim Zurückziehen in einer Position innerhalb des Gehäuses abschirmt.

Die WO 97/02060 beschreibt eine automatische Kanülenrückzugsvorrichtung für Injektionsspritzen. Sie besteht aus einer Kanüle mit einer Hohlnadel und einem Gehäuse sowie einer Einrichtung, mit der die Hohlnadel nach ihrer Benutzung in das Gehäuse zurückgezogen werden kann. Die Einrichtung weist dafür eine Umschaltvorrichtung auf, die nach der Benutzung der Hohlnadel für deren Rückzug schaltbar ist.

Die WO 2009/046560 A2 betrifft eine Sicherheitsanordnung für eine Verweilkanüle. Die Verweilkanüle besitzt eine Kanüle und einen Katheter. Die Kanüle weist einen Sockel auf und der Katheter weist einen Adapter auf. Dabei ist die Kanüle in den Katheter derart einschiebbar ausgebildet, dass der Sockel im Adapter liegt und die Kanüle betriebsfertig durch den Katheter hindurch ragt. Es ist ein Schutzelement für die verletzungssichere Abdeckung der Kanüle vorgesehen, das entlang der Kanüle zwischen einer Betriebslage mit freier Kanüle und einer die Kanülenspitze abdeckenden Sicherheitslage verschoben werden kann. Dabei ist das Schutzelement über ein Zugglied gegenüber dem Sockel der Kanüle festgelegt, derart, dass es im gespannten Zustand des Zugglieds die Sicherheitslage einnimmt und dass das Sicherheitselement eine Verriegelungsanordnung zu dessen Verriegelung in Sicherheitslage aufweist.

Die EP 2 319 556 B1 beschreibt einen Nadelspitzenschutz für Subkutaninjektionen. Dieser besteht aus einem Nadelschutz, der verschiebbar auf der Nadel angebracht ist, wobei der Nadelschutz einen beweglichen Nadelfänger umfasst, der in Richtung der Nadel vorgespannt ist. Der Nadelfänger des Nadelschutzes bewegt sich über das spitze distale Ende der Nadel vorwärts und deckt es dadurch ab, wenn der Nadelschutz nach vorne in die Nähe des spitzen distalen Endes der Nadel gedrückt wird. Die Vorwärtsbewegung des Nadelschutzes längs der Nadel wird durch ein Begrenzungsmittel begrenzt, welches eine Anhängevorrichtung umfasst. Die Nadelschutzvorrichtung besitzt einen Kopplungsmechanismus, der eine mechanische Trennung der Nadelschutzanordnung vom Katheteransatz verhindert, bis das spitze distale Ende sicher von dem Nadelfänger abgedeckt ist.

Nachteilig an den bekannten Venenverweilkanülen ist, dass der flexible Katheter durch die Hohlnadel, insbesondere durch die Schneidekante der Hohlnadel beschädigt werden kann. Diese Beschädigung kann insbesondere nach dem Zurückziehen der Hohlnadel erfolgen, wenn diese wieder vorgeschoben wird. Vor dem Wiedervorschieben wird ausdrücklich gewarnt, beispielsweise heißt es in einer Anleitung von BBraun (HD.02.12.15/1 Nr. 6032220): "Achtung. Schieben Sie die Stahlkanüle nach dem ersten Zurückziehen nicht erneut in den Katheter vor, der Katheter könnte beschädigt oder sogar abgeschert werden - dies kann eine Embolie verursachen." Dabei kann es aus mehreren Gründen zu einem Wiedervorschieben der Hohlnadel kommen. Beispielsweise kann dies auf Grund mangelnder Kenntnis des Anwenders über die korrekte Produktanwendung beziehungsweise Unachtsamkeit des Anwenders geschehen. Weitere Gründe können die Bewegungen eines unruhigen Patienten oder Erschütterungen, beispielsweise durch den Einsatz in einem fahrenden Rettungswagen, sein.

Ein beschädigter Katheter kann zu Irritationen des Gewebes des Patienten oder der Gefäßinnenwand der punktierten Vene führen. Wird die Hohlnadel beim Zurückziehen wieder vorgeschoben, während sich die Spitze der Hohlnadel innerhalb des flexiblen Katheters befindet, so kann der flexible Katheter von der Spitze durchstochen werden. Durch diese Perforation des Katheters kann die zu injizierende Flüssigkeit in das Gewebe des Patienten eindringen und dort Schäden, beispielsweise Entzündungen und Nekrosen, verursachen. Ein solcher durchstochener Katheter muss daher entsorgt werden und der Venenzugang muss erneuert werden, was einen höheren Zeitaufwand bei der Behandlung des Patienten bedeutet. Auch können sich Teile des flexiblen Katheters durch Kontakt mit der Spitze der Hohlnadel lösen und in den Körper des Patienten gelangen. Dort können sie eine Fremdkörperreaktion im Gewebe des Patienten verursachen. Besonders gefährlich für den Patienten ist das Eindringen eines solchen Teiles in die Blutbahn, da dies eine Embolie auslösen kann. Zu den Risiken des Einführens von Partikeln ins Blutsystems sei auf die Seite https://www.bbraunforsafety.com/en/particulatecontamination.html verwiesen.

Entsprechend sind Schutzmechanismen für den flexiblen Katheter einer Venenverweilkanüle bekannt.

So betrifft die DE 20 2018 101 646 U1 eine Venenverweilkanüle zur Applikation an einem Lebewesen mit einem Venenkatheter, wobei eine Punktionsnadel längsverschieblich in dem Venenkatheter führbar ist. Damit der flexible Teil des Venenkatheters durch Kontakt mit der Punktionsnadel nicht durchstochen werden kann, ist wenigstens der Teil des Venenkatheters, der zum Verweilen im Lebewesen eingerichtet ist, über seine gesamte Länge, in der die Punktionsnadel längsverschieblich führbar ist, aus Metall oder hat eine Metallschicht. Nachteilig hieran ist, dass der flexible Katheter aufwendig mit einer Metallschicht überzogen werden muss.

Es sind weiterhin Punktionsvorrichtungen bekannt. Die Seldinger-Punktionstechnik wird beispielsweise für das Legen zentraler Venenzugänge oder auch für arterielle Zugänge angewendet. Der flexible Seldingerdraht wird durch die Punktionsnadel bis ins Gefäß vorgeschoben. Der Seldingerdraht wird dann durch Festhalten oder Druck auf das Gefäß in seiner Position fixiert. Anschließend wird die Punktionsnadel vorsichtig herausgezogen. Nun wird ein Dilatator auf den Seldingerdraht gesteckt, vorgeschoben und die Hautschichten sowie die Gefäßwand aufgedehnt. Der Dilatator wird bei weiterhin fixiertem Seldingerdraht wieder zurückgezogen. Danach wird der Katheter auf den Seldingerdraht aufgesteckt und über diesen als Leitschiene in das Gefäßlumen vorgeschoben. Bei dieser Prozedur ist nicht auszuschließen, dass der Seldingerdraht bezüglich der Punktionsnadel zurückgezogen wird. Da die Spitze der Punktionsnadel sehr scharf ist, kann es dadurch zu Beschädigungen des Seldingerdrahtes, beispielsweise zum Abscheren eines Drahtstückes, kommen. Dieses kann dann ins venöse oder arterielle Blutsystem gelangen und schwere Schäden verursachen.

Aus der US 2013/0178711 A1 ist eine Vorrichtung und ein Verfahren für endovaskuläre Therapien bekannt, einschließlich der Erleichterung der Schaffung eines Gefäßzugangs, der Platzierung endovaskulärer Schleusen, der Lokalisierung der Katheterspitze und der Behandlung eines Gefäßverschlusses.

Die US 2019/0351196 A1 beschreibt ein Einführungswerkzeug zum Einführen eines Katheters in den Körper eines Patienten mit einem Gehäuse, in dem zumindest ein Teil des Katheters anfänglich angeordnet ist, einer Hohlnadel, die sich distal von dem Gehäuse erstreckt, wobei der Katheter über der Nadel angeordnet ist, und einem Führungsdraht, der in der Nadel angeordnet ist.

Die US 2017/0239443 A1 beschreibt eine Katheterbaugruppe mit einem Katheterkörper, einem Septum und einem Septumhalter, wobei der Katheterkörper ein distales Ende, ein proximales Ende und eine Innenwand aufweist, die einen inneren Fluiddurchgang dazwischen definiert.

Aus der US 2013/0178711 A1 ist eine Vorrichtung und ein Verfahren für die endovaskuläre Therapie, einschließlich der Erleichterung des Gefäßzugangs, die Platzierung endovaskulärer Schleusen, die Lokalisierung von Katheterspitzen und die Verabreichung von Gefäßverschlüssen bekannt.

Der vorliegenden Erfindung liegt die Aufgabe zugrunde, die bekannten Verweilkanülen zu optimieren.

Diese Aufgabe wird bei einer Verweilkanüle gemäß dem Oberbegriff des Anspruchs 1 dadurch gelöst, dass eine Verformung und/oder Beschädigung der Verweilkanüle durch eine Sperrvorrichtung während der Lagerung vermieden wird, wobei die Sperrvorrichtung durch einen Feder-Klemmkonus mit einer Feder und zwei Klemmflügeln ausgebildet ist, wobei die Feder durch ein Führungselement geführt wird und die Sperrung der proximalen und distalen Bewegung des Katheters beim Transport und/oder der Lagerung durch die Sperrvorrichtung mittels einer proximalen Transportsicherungsvorrichtung und einer distalen Transportsicherung über Stifte, die von beiden Seiten gegen die Feder drücken, vermieden wird.

Die als Punktionsvorrichtung dienende Verweilkanüle kann beispielsweise zur Durchführung der Seldinger-Punktionstechnik ausgestaltet sein. Dabei entspricht der Einsatz dem Seldingerdraht und die Hohlnadel einer Punktionskanüle, in welche der Seldingerdraht eingeführt wird. Vorteilhaft erlaubt die Sperrvorrichtung ein Einführen des Seldingerdrahtes in die Punktionsnadel und ein leichtes Vorschieben des Seldingerdrahtes innerhalb der Punktionsnadel, ein Zurückschieben des Seldingerdrahtes wird jedoch blockiert. Dabei kann die Sperrvorrichtung fest in den Anschlusskonus der Punktionsnadel eingebaut sein. Bei einer eventuell erforderlichen erneuten Punktion kann die Kombination aus Seldingerdraht und Punktionsnadel aus dem Patienten entfernt werden. Anschließend kann der Seldingerdraht nach vorne (proximal) aus der Punktionsnadel herausgezogen werden und kann wieder, eventuell unter der Zuhilfenahme einer Einführhilfe, von hinten (distal) in die Nadel eingeführt werden.

Die Verweilkanüle gemäß der vorliegenden Erfindung ist vorzugsweise ein flexibler Katheter, insbesondere ein Periduralkatheter oder ein Seldingerdraht.

Ein solcher flexibler Katheter kann beispielsweise ein Periduralkatheter sein. Ein Zurückziehen des Periduralkatheters wird dabei vorteilhaft durch die Sperrvorrichtung blockiert. Auf diese Weise wird eine Beschädigung, beispielsweise durch Abscherung, des Periduralkatheters verhindert.

Beispielsweise läuft beim Periduralkatheter der flexible Katheter innerhalb der Hohlnadel. Um ein Zurückziehen des flexiblen Katheters innerhalb der Hohlnadel zu verhindern, ist die Sperrvorrichtung ein Feder-Klemmkonus, welcher die Bewegung des flexiblen Katheters innerhalb der Hohlnadel in distaler Richtung blockiert. Auf diese Weise wird eine Beschädigung, beispielsweise durch Abscherung, des Periduralkatheters verhindert. Die Oberflächen des Periduralkatheters und die Kontaktfläche des Feder-Klemmkonus können dabei mikroskopisch oder im Nanobereich so aufeinander abgestimmt werden, dass eine gute Klemmwirkung erreicht wird.

Eine vorteilhafte Ausgestaltung der Erfindung besteht darin, dass die Schraubverbindung mittels der Überwurfmutter zwischen der Hohlnadel und dem Gehäuse nicht mehr lösbar ist.

Weiterhin ist im Rahmen der Erfindung vorgesehen, dass der Feder-Klemmkonus der Sperrvorrichtung je nach Status der Anwendung automatisch die richtige Einstellung/Schaltstellung einnimmt

Die Aufgabe wird auch bei einer Verweilkanüle gemäß der Erfindung dadurch gelöst, dass die Sperrvorrichtung ein Wiederzurückschieben des Katheters in distaler Richtung verhindert.

Durch die Blockierung der Längsverschiebung in proximaler Richtung durch die form- und/oder kraftschlüssige Verbindung zwischen der Sperrvorrichtung und der Hohlnadel wird eine Beschädigung des Katheters vorteilhaft verhindert. Dabei kann die Hohlnadel, nachdem sie die zu punktierende Vene punktiert hat und der Katheter in die Vene eingelegt ist, durch eine Längsverschiebung in distaler Richtung aus dem Katheter zurückgezogen und damit herausgezogen werden.

Schließlich ist auch vorgesehen, dass der Feder-Klemmkonus der Sperrvorrichtung je nach Status der Anwendung automatisch die richtige Einstellung/Schaltstellung einnimmt

Grundsätzlich unterscheidet sich die Bedienung der erfindungsgemäßen Verweilkanüle nicht von den etablierten Produkten. Ein Anlernen oder Umlernen der Anwender ist daher vorteilhaft nicht notwendig. Dies erhöht die Akzeptanz bei den Anwendern.

Nachfolgend werden Ausführungsbeispiele der Erfindung anhand von Zeichnungen näher beschrieben.

Es zeigen
- Fig. 1: eine nicht erfindungsgemäße Verweilkanüle im Längsschnitt,
- Fig. 2: die gleiche Verweilkanüle im Längsschnitt,
- Fig. 3: die gleiche Verweilkanüle im Längsschnitt,
- Fig. 4: eine weitere nicht erfindungsgemäße Verweilkanüle im Längsschnitt,
- Fig. 5: einen erfindungsgemäßen Käfig in der Draufsicht vor dem Zusammenbau,
- Fig. 6: eine nicht erfindungsgemäße Hohlnadel in der Seitenansicht und im Querschnitt,
- Fig. 7: einen nicht erfindungsgemäßen Käfig im Querschnitt,
- Fig. 8: einen weiteren nicht erfindungsgemäßen Käfig im Querschnitt,
- Fig. 9: eine erfindungsgemäße Verweilkanüle im Längsschnitt,
- Fig. 10: die Verweilkanüle aus Fig. 9 in einem anderen Anwendungsschritt im Längsschnitt,
- Fig. 11: die Verweilkanüle aus Fig. 9 in einem weiteren Anwendungsschritt im Längsschnitt,
- Fig. 12: die Verweilkanüle aus Fig. 9 in einem weiteren Anwendungsschritt im Längsschnitt,
- Fig. 13: eine weitere erfindungsgemäße Verweilkanüle im Längsschnitt,
- Fig. 14: eine nicht erfindungsgemäße Verweilkanüle im Längsschnitt.

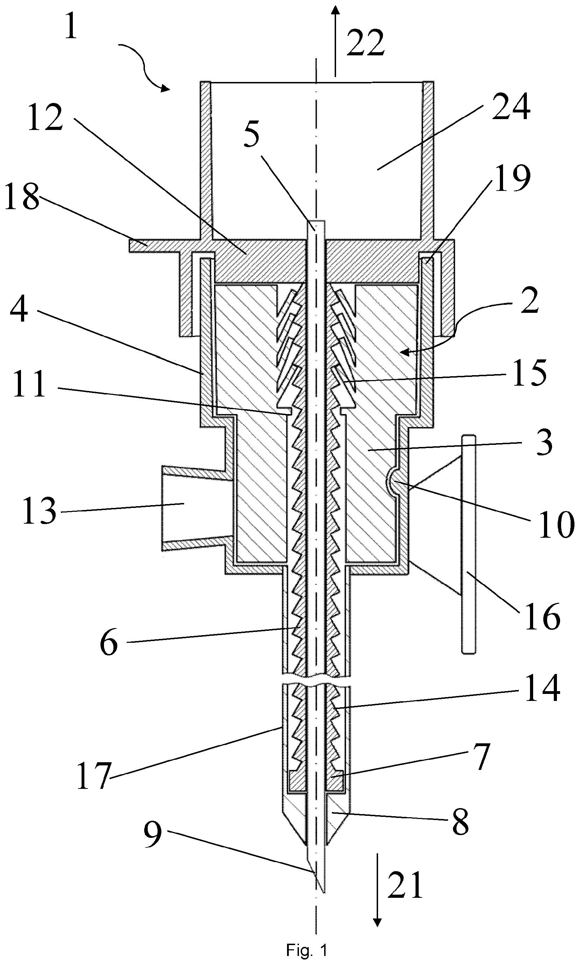

Fig. 1 stellt eine Ausführungsform einer nicht erfindungsgemäßen Verweilkanüle 1 dar. Die Hohlnadel 5 ist hierbei maximal in den flexiblen Katheter 17 eingeschoben. Allein der vordere Teil der Hohlnadel 5 mit der Nadelspitze 9 ragt aus dem Katheter 17 hervor. Die Nadelspitze 9 besitzt eine Schnittkante, mit der die Haut und die Vene des Patienten punktiert werden können.

Der Katheter 17 ist an seinem distalen Ende mit einem Gehäuse 4 verbunden. Zur Zuführung von Medikamenten besitzt das Gehäuse 4 einen Zuspritzport 13. Das Gehäuse 4 besitzt eine Anschlusseinheit 19, über die beispielsweise Injektionssysteme mit dem Katheter 17 verbunden werden können. An dem Gehäuse 4 ist zusätzlich eine Stabilisierungsplattform 16 zum sicheren Fixieren des Katheters 17 am Patienten angebracht.

Nach dem erfolgreichen Punktieren der Vene tritt Blut aus dem distalen Ende der Hohlnadel 5 in die Blutrückflusskammer 24 aus. Danach wird der Katheter 17 weiter in die Vene vorgeschoben. Die Katheterspitze 8 liegt dann in der Vene des Patienten. Der geringere Innendurchmesser des Katheters 17 bezüglich seiner Katheterspitze 8 verleiht dieser in Verbindung mit dem Abschlussring 7 eine höhere Stabilität. Der Abschlussring 7 liegt auf der Auflagefläche der Katheterspitze 8 auf und erhöht die Stabilität der Verweilkanüle 1 bei vollständig in den Katheter 17 eingeschobener Hohlnadel 5. Die Auflagefläche der Katheterspitze 8 verhindert zusammen mit dem Abschlussring 7, dass es bei der Punktion zu einer Einstauchung des Katheters 17 kommt. Diese Gefahr besteht, wenn die zu punktierende Haut einen hohen mechanischen Widerstand aufweist. Der Abschlussring 7 läuft dichtend an der Innenwand des Katheters 17 entlang und verhindert das Eindringen von Blut in den Raum distal von ihm zwischen der Innenwand des Katheters 17 und der Außenwand der Hohlnadel 5. Die entsprechende Nadeloberfläche, der Käfig 3 und die Sperrvorrichtung 2 werden nicht mit Blut kontaminiert. Somit kann die Funktion der Sperrvorrichtung 2 nicht durch Blut beeinträchtigt werden und die Hohlnadel 5 stellt Dank der sauberen Oberfläche nach dem Herausziehen eine deutlich geringere Kontaminationsgefahr dar. Liegt der Katheter 17 an seiner vorgesehenen Position in der Vene des Patienten, so kann die Hohlnadel 5 über den Griff 18 an dem Nadelgriffstück 12 aus dem Katheter 17 in distaler Richtung 22 herausgezogen werden.

Die Sperrvorrichtung 2 verhindert dabei ein Wiedervorschieben der Hohlnadel 5 in proximaler Richtung 21. Die Sperrvorrichtung 2 weist Lamellen 15 auf, die mit Zähnen 14 des Kunststoffüberzugs 6 der Hohlnadel 5 bei Bewegung der Hohlnadel 5 innerhalb des Katheters 17 in proximaler Richtung 21 eine Rastverbindung eingehen und die Bewegung dadurch blockieren. Die Sperrvorrichtung 2 ist dabei innerhalb des Gehäuses 4 des Katheters 17 durch eine Rastnase 10 lösbar fixiert. Dabei ist die Kraft, die zum Lösen der Sperrvorrichtung 2 mit dem Käfig 3 von dem Gehäuse 4 nötig ist, größer als die Kraft, die durch das Herausziehen der Hohlnadel 5 aus dem Katheter 17 auf die Sperrvorrichtung 2 mit dem Käfig 3 wirkt, zumindest bis der Abschlussring 7 der Hohlnadel 5 an die Auflagefläche 11 des Käfigs 3 anstößt.

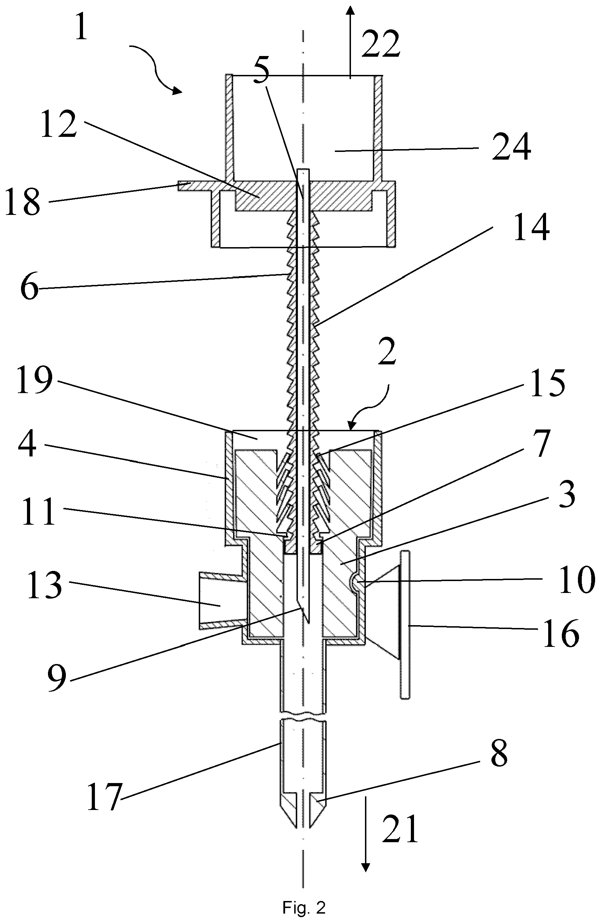

In Fig. 2 ist die Hohlnadel 5 bis zu dem Punkt aus dem Katheter 17 herausgezogen, an dem die Auflagefläche des Käfigs 11 und die Auflagefläche des Abschlussrings 7 zusammentreffen. Durch die Auflagefläche des Käfigs 11 und die Auflagefläche des Abschlussrings 7 kann eine Kraft, die größer als die Kraft, die die Sperrvorrichtung 2 innerhalb des Gehäuses 4 hält, ist, übertragen werden. Somit wird durch das weitere Herausziehen der Hohlnadel 5 aus dem Katheter 17 die Rastverbindung durch die Rastnase 10 gelöst und die Sperrvorrichtung 2 mit dem Käfig 3 zusammen mit der der Hohlnadel 5 aus dem Gehäuse 4 gezogen.

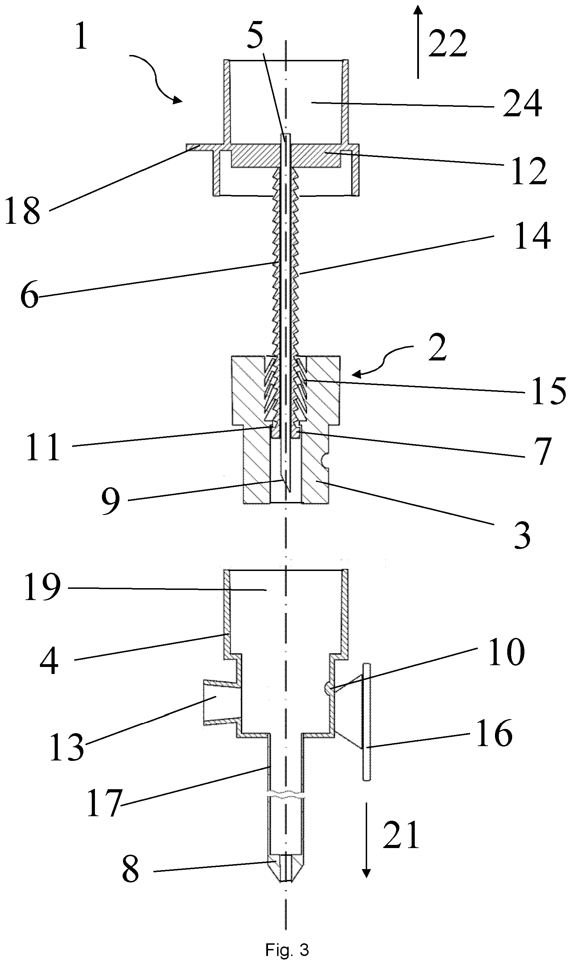

In Fig. 3 ist die vollständig aus dem Katheter 17 herausgezogene Hohlnadel 5 gezeigt. Der Käfig 3 überdeckt dabei die Nadelspitze 9 und dient als Stichschutz. Durch die Auflagefläche des Käfigs 11 in Verbindung mit dem Abschlussring 7 und der Sperrvorrichtung 2 lässt sich der Käfig nicht mehr von der Hohlnadel 5 entfernen. Ein Wiederverwenden der Hohlnadel 5 ist damit nicht möglich. Durch den Käfig 3 kann die Hohlnadel 5 sicher entsorgt werden. Da das Verriegeln und Überdecken der Nadelspitze 9 automatisch erfolgt, handelt es sich um ein passives Sicherheitsgerät.

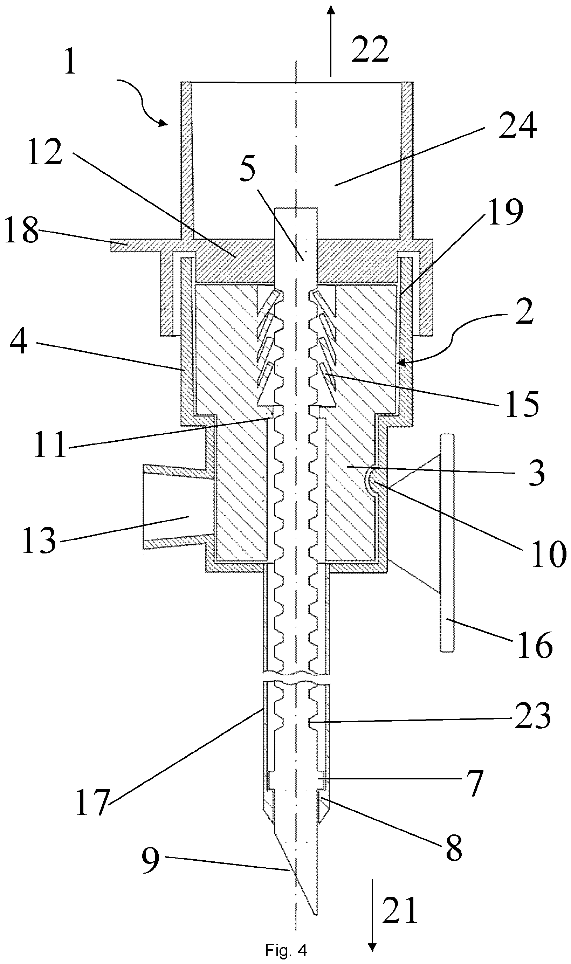

In Fig. 4 ist eine alternative Ausgestaltung einer nicht erfindungsgemäßen Verweilkanüle 1 dargestellt. Anstatt eines Kunststoffüberzugs 6 ist die Hohlnadel 5 an ihrer Oberfläche mit Vertiefungen 23 versehen. Diese Vertiefungen 23 können mechanisch oder durch einen Laser bei der Herstellung der Hohlnadel 5 erzeugt werden. Der Abschlussring 7 der Hohlnadel kann dabei durch ein Auseinanderquetschen der Hohlnadel 5 im Querschnitt oder durch ein Umspritzen der Nadel in Form eines Kunststoffrings erzeugt werden. Um ein Wiedervorschieben der Hohlnadel 5 in proximaler Richtung 21 zu verhindern, weist die Verweilkanüle 1 eine Sperrvorrichtung 2 auf. Die Sperrvorrichtung 2 weist Lamellen 15 auf, die mit den Vertiefungen 23 der Hohlnadel 5 bei Bewegung der Hohlnadel 5 innerhalb des Katheters 17 in proximaler Richtung 21 eine Rastverbindung eingehen und die Bewegung dadurch blockieren.

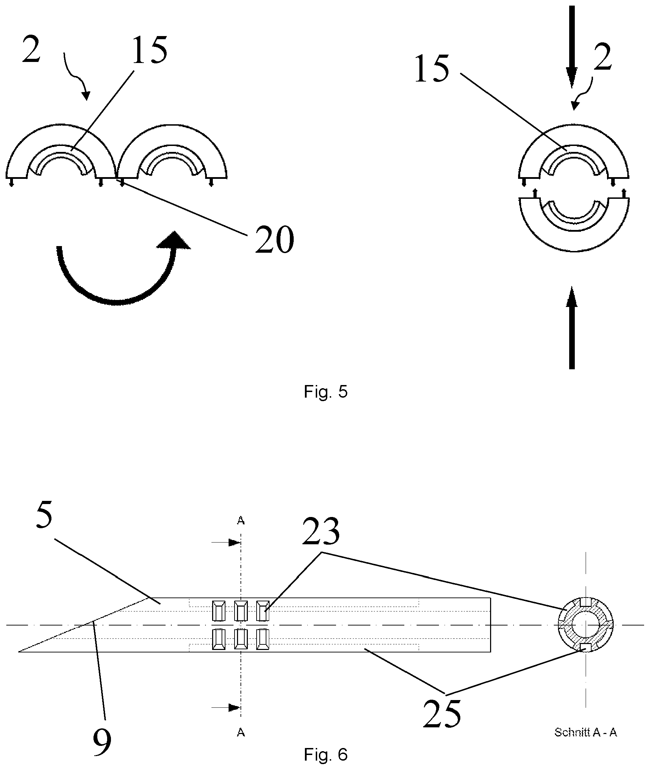

Fig. 5 zeigt eine Draufsicht auf die Sperrvorrichtung 2 vor dem Zusammenbau. Die Lamellen 15 bilden mit dem Kunststoffüberzug 6 eine Rastverbindung und verhindern im zusammengebauten Zustand das Wiedervorschieben der Hohlnadel 5.

Wie auf der linken Seite der Figur zu sehen ist, kann die Einheit aus Sperrvorrichtung 2 und Käfig 3 aus zwei Halbschalen, die mit einem Filmscharnier 20 verbunden sind, mittels Einrastverbindung zusammengebaut werden. Auf der rechten Seite der Figur sind zwei nicht durch ein Filmscharnier 20 verbundenen Halbschalen der Einheit aus Sperrvorrichtung 2 und Käfig 3 dargestellt, welche durch eine Einrastverbindung verbunden werden können. Das Verbinden der Halbschalen kann auch durch Schweißen oder Kleben erfolgen.

In Fig. 6 ist eine Hohlnadel 5 mit Nadelspitze 9 dargestellt. Die Hohlnadel 5 besitzt Vertiefungen 23, die mechanisch oder durch einen Laser bei der Herstellung der Hohlnadel 5 erzeugt werden können. Die Sperrvorrichtung 2 kann mit den Vertiefungen 23 der Hohlnadel 5 bei Bewegung der Hohlnadel 5 innerhalb des Katheters 17 in proximaler Richtung 21 eine Rastverbindung eingehen und die Bewegung dadurch blockieren. Die Hohlnadel 5 weist eine Längsnut 25 auf, die zusammen mit Führungsnasen der Sperrvorrichtung ein Drehen der Hohlnadel 5 in dem Katheter 17 und somit das Beschädigen des Katheters 17 durch die Nadelspitze 9 verhindert. Die Längsnut 25 kann dabei durch den Kunststoffüberzug 6 der Hohlnadel 5 oder durch mechanische Bearbeitung der Hohlnadel 5 hergestellt werden. Die Längsnut 25 kann auch durch die Bearbeitung mittels eines Lasers entstehen.

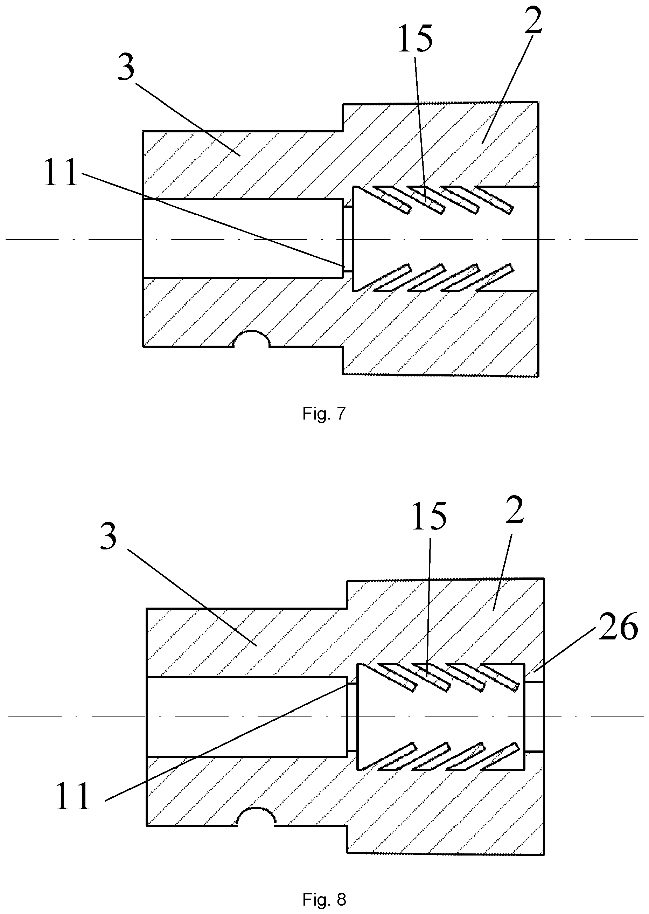

In Fig. 7 ist eine Einheit aus Sperrvorrichtung 2 und Käfig 3 dargestellt. Die Sperrvorrichtung 2 weist Lamellen 15 auf, die die Bewegung der Hohlnadel 5 innerhalb des Katheters 17 in proximaler Richtung 21 durch eine Rastverbindung blockieren. Die Hohlnadel 5 kann bis zu dem Punkt aus dem Katheter 17 herausgezogen, an dem die Auflagefläche des Käfigs 11 und die Auflagefläche des Abschlussrings 7 zusammentreffen. Der Käfig 3 überdeckt dabei die Nadelspitze 9 der herausgezogenen Hohlnadel 5 und dient als Stichschutz. Durch die Auflagefläche des Käfigs 11 in Verbindung mit dem Abschlussring 7 und die Sperrvorrichtung 2 lässt sich der Käfig nicht mehr von der Hohlnadel 5 entfernen.

Wie in Fig. 8 zu sehen ist, kann die Sperrvorrichtung 2 zusätzlich einen Begrenzungsring 26 aufweisen. Der Begrenzungsring 26 verbessert dabei die Führung der Hohlnadel 5 innerhalb der Sperrvorrichtung 2.

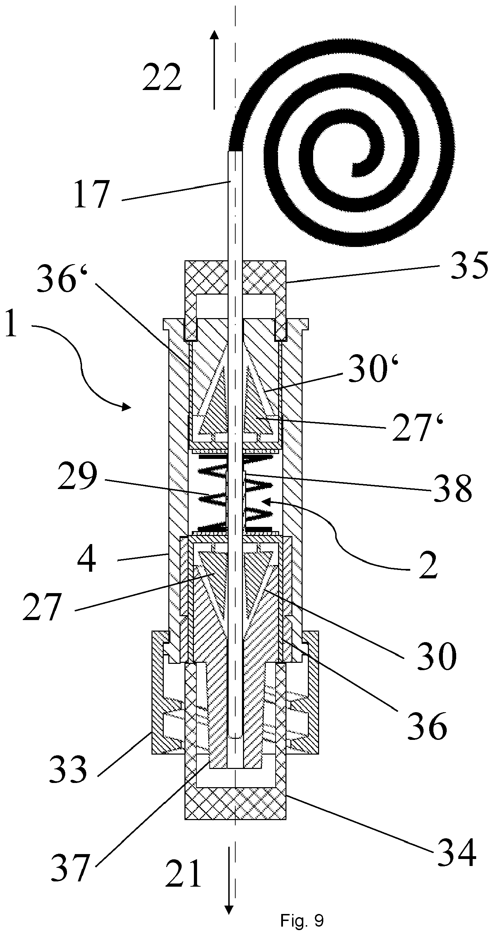

Die Figuren 9 bis 12 zeigen eine Ausführungsform der erfindungsgemäßen Verweilkanüle 1 in verschiedenen Stadien der Anwendung. Dabei handelt es sich um einen Periduralkatheter.

Der Periduralkatheter ist in Fig. 9 in dem Zustand der Lagerung und/oder des Transportes dargestellt. Die Hohlnadel 5 zur Punktion des Patienten ist dabei nicht dargestellt. Die Hohlnadel 5 kann mittels einer Überwurfmutter 33 mit dem Gehäuse 4 verbunden werden. Der Katheter 17 verläuft dabei frei durch das Gehäuse 4. Auf den Katheter 17 wirken keine Klemmkräfte. Dadurch wird eine Verformung und/oder Beschädigung des Katheters 17 durch die Sperrvorrichtung 2 während der Lagerung und des Transportes vermieden. Die Sperrvorrichtung 2 ist als Feder-Klemmkonus mit einer Feder 29 und zwei Klemmflügeln 27 und 27' ausgestaltet. Die Feder 29 wird durch ein Führungselement 38 geführt. Das Führungselement 38 schützt den Katheter 17 vor Beschädigungen. Die Sperrung der proximalen und distalen Bewegung des Katheters 17 beim Transport und/oder der Lagerung durch die Sperrvorrichtung 2 wird mittels der proximalen Transportsicherungsvorrichtung 34 und der distalen Transportsicherungsvorrichtung 35 über Stifte 36 und 36' vermieden. Die Stifte 36 und 36' drücken dabei von beiden Seiten gegen die Feder 29. Dadurch können die Klemmflügel 27 und 27' nicht an ihre Anlageflächen 30 und 30' anliegen. Es werden somit bei der Bewegung des Katheters 17 innerhalb des Gehäuses 4 keine Klemmkräfte auf den Katheter 17 ausgeübt.

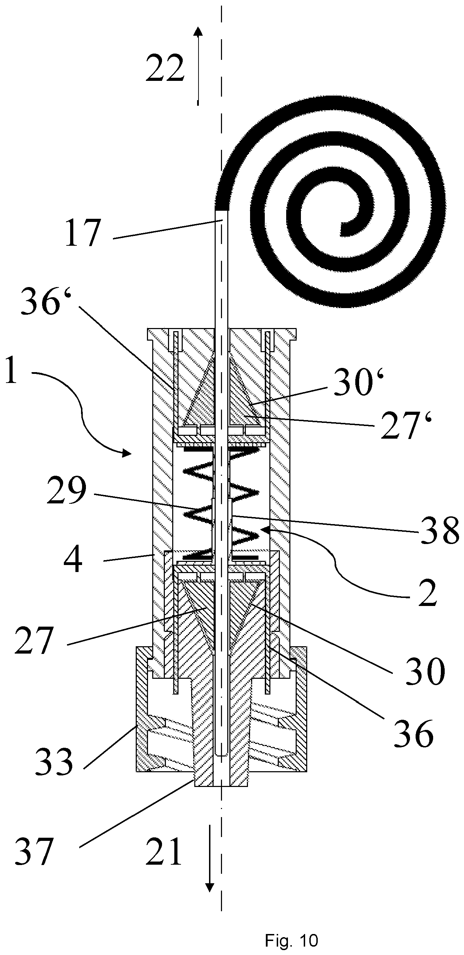

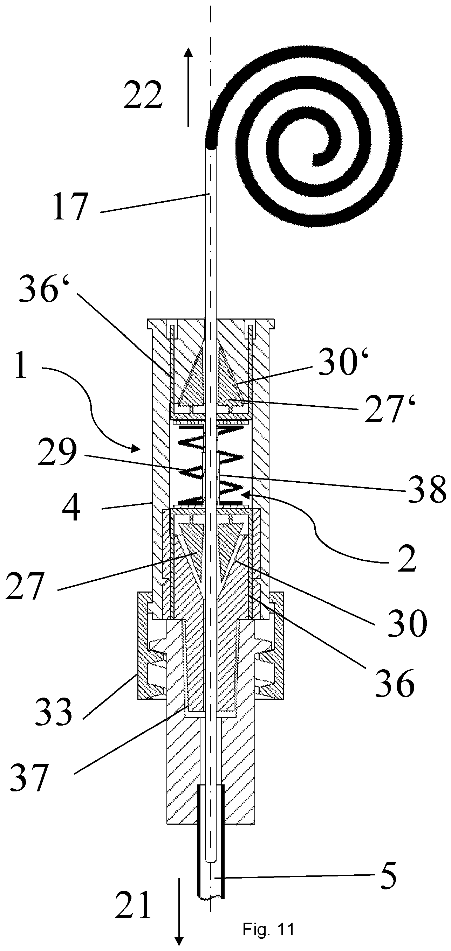

Vor der Anwendung des Periduralkatheters werden die proximale Transportsicherungsvorrichtung 34 und die distale Transportsicherungsvorrichtung 35 entfernt. Dies ist in Fig. 10 dargestellt. Dabei kann die Verpackung des Periduralkatheters so gestaltet werden, dass beim Entnehmen des Periduralkatheters aus der Verpackung die proximale Transportsicherungsvorrichtung 34 und die distale Transportsicherungsvorrichtung 35 konstruktionsbedingt zwangsweise entfernt werden. Der Katheter 17 wird nun durch die Sperrvorrichtung sowohl in der Bewegung in proximaler Richtung 21 und in distaler Richtung gehindert. Vorteilhaft kann der Katheter 17 dadurch nicht aus dem Gehäuse 4 gezogen werden. Nach erfolgreicher Punktion des Patienten mit der Hohlnadel/Punktionsnadel 5 wird anschließend das Gehäuse 4 mit dieser mittels der Überwurfmutter 33 verbunden, wie in Fig. 11 gezeigt. Diese Schraubverbindung ist nicht mehr lösbar. Statt der Schraubverbindung kann auch eine Steckverbindung realisiert werden. Dabei wird das Gehäuse 4 auf die Hohlnadel 5 gesteckt und rastet mit einem deutlich hör- und fühlbaren "Click" ein. Auch diese Steckverbindung ist nicht wieder lösbar. Die Hohlnadel 5 und das Gehäuse 4 können nicht gegeneinander verdreht werden. Möglich ist auch eine Version, bei der ein Verdrehen möglich ist.

Durch diese Schraub- oder Steckverbindung wird über den Stift 36 die Feder 29 der Sperrvorrichtung 2 zusammengedrückt. Der Katheter 17 kann innerhalb des Gehäuses 4 nur in proximaler Richtung 21 verschoben werden. Bei Bewegung des Katheters 17 in distaler Richtung 22 werden die Klemmflügel 27' gegen die Anlagefläche 30' gedrückt. Dadurch kommt es zu einer Klemmwirkung der Sperrvorrichtung 2 gegen den Katheter 17. Nach dem erfolgreichen Punktieren des Patienten mit der Hohlnadel 5 wird der Katheter 17 in proximaler Richtung 21 verschoben, um den Katheter 17 durch die Hohlnadel 5 in den Patienten einzuführen. Der Katheter 17 wird dabei über die Einführhilfe 37 in die Hohlnadel 5 geführt.

Die Sperrvorrichtung 2 verhindert dabei ein Wiederzurückschieben des Katheters 17 in distaler Richtung 22. Die Klemmflügel 27' gehen mit dem Katheter 17 bei Bewegung des Katheters 17 in distaler Richtung 22 eine kraftschlüssige Verbindung ein und die Bewegung wird dadurch blockiert. Vorteilhaft wird ein Verschieben des Katheters 17 in distaler Richtung 22 und damit eine mögliche Beschädigung des Katheters 17 durch die Hohlnadel 5 verhindert.

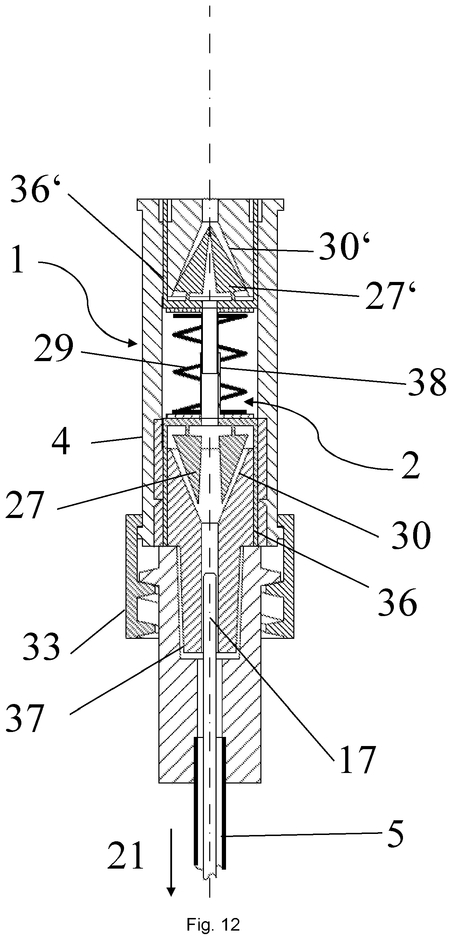

Befindet sich der Katheter 17 an seiner vorgesehenen Position im Patienten, kann die Hohlnadel 5 zusammen mit dem damit verbundenen Gehäuse 4 aus dem Patienten herausgezogen werden. Dies ist in Fig. 12 dargestellt. Damit der Katheter 17 bei diesem Herausziehen der Hohlnadel 5 an seiner vorgesehenen Position verbleibt, kann der Katheter 17 durch die Sperrvorrichtung 2 in proximaler Richtung 21 bewegt werden. Bei der Bewegung des Katheters 17 in proximaler Richtung 21 werden die Klemmflügel 27 durch die Stifte 36 gegen die Feder 29 gedrückt. Es entsteht keine Klemmwirkung durch die Sperrvorrichtung 2 gegen die Bewegung des Katheters 17. Vorteilhaft nimmt der Feder-Klemmkonus der Sperrvorrichtung 2 je nach Status der Anwendung automatisch die richtige Einstellung/Schaltstellung ein.

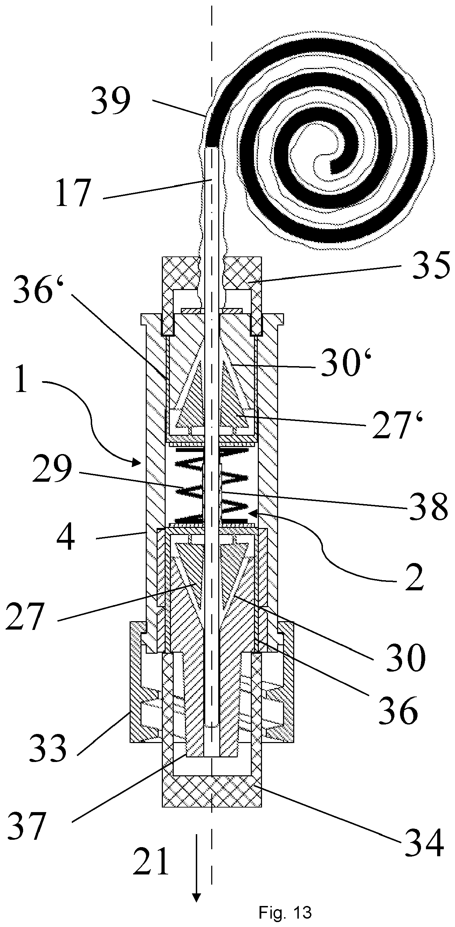

In Fig. 13 ist eine weitere Ausführungsform der erfindungsgemäßen Verweilkanüle 1 als Periduralkatheter dargestellt. Die Verweilkanüle 1 zeigt dabei alle Eigenschaften der Verweilkanüle aus Figur 9. Zusätzlich weist die Verweilkanüle 1 eine Schutzhülle 39 auf. Die Schutzhülle 39 umhüllt den Katheter 17. Die Schutzhülle 39 schützt den Katheter 17 vorteilhaft vor Verunreinigung.

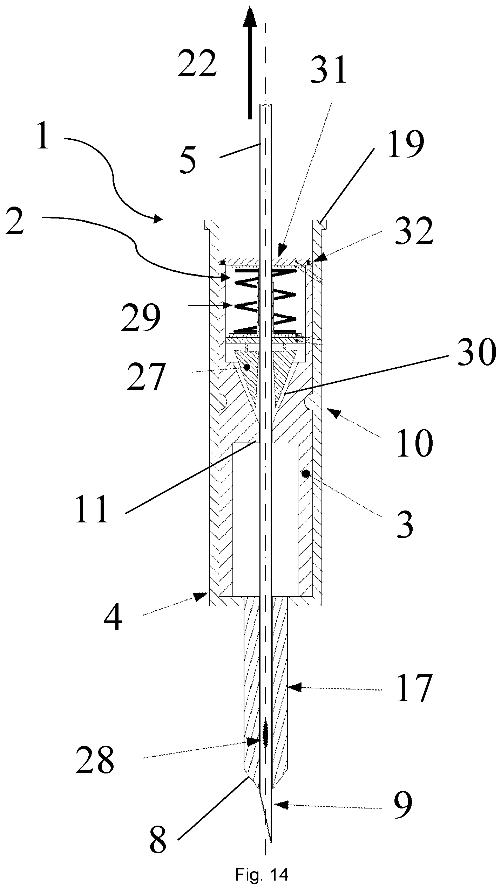

Fig. 14 stellt eine weitere Ausführungsform einer nicht erfindungsgemäßen Verweilkanüle 1 dar. Dabei handelt es sich um eine periphere Venenverweilkanüle. Die Hohlnadel 5 ist hierbei maximal in den flexiblen Katheter 17 eingeschoben. Allein der vordere Teil der Hohlnadel 5 mit der Nadelspitze 9 ragt aus dem Katheter 17 hervor. Die Nadelspitze 9 besitzt eine Schnittkante, mit der die Haut und die Vene des Patienten punktiert werden können.

Der Katheter 17 ist an seinem distalen Ende mit einem Gehäuse 4 verbunden. Das Gehäuse 4 besitzt eine Anschlusseinheit 19, über die beispielsweise Injektionssysteme oder Infusionssysteme mit dem Katheter 17 verbunden werden können.

Nach dem erfolgreichen Punktieren der Vene tritt Blut aus dem distalen Ende der Hohlnadel 5 aus. Danach wird der Katheter 17 weiter in die Vene vorgeschoben. Die Katheterspitze 8 liegt dann in der Vene des Patienten. Liegt der Katheter 17 an seiner vorgesehenen Position in der Vene des Patienten, so kann die Hohlnadel 5 in distaler Richtung 22 herausgezogen werden.

Die Sperrvorrichtung 2 verhindert dabei ein Wiedervorschieben der Hohlnadel 5 in proximaler Richtung 21. Die Sperrvorrichtung 2 ist als Feder-Klemmkonus ausgestaltet. Die Sperrvorrichtung 2 weist Klemmflügel 27 auf, die mit der Hohlnadel 5 bei Bewegung der Hohlnadel 5 innerhalb des Katheters 17 in proximaler Richtung 21 eine kraftschlüssige Verbindung eingehen und die Bewegung dadurch blockieren. Bei der Bewegung der Hohlnadel 5 in proximaler Richtung 21 werden die Klemmflügel 27 durch die Hohlnadel 5 und eine Feder 29 gegen eine Anlagefläche 30 gedrückt. Dadurch entsteht eine Klemmwirkung durch die Sperrvorrichtung 2 gegen die Bewegung der Hohlnadel 5. Dabei kann die Oberfläche der Hohlnadel 5 physikalisch und/oder chemisch so behandelt werden, dass sie mit den Klemmflügeln 27 eine sichere Hemmung garantieren. Dabei werden die Klemmflügel 27 durch die Feder 29 in Richtung der Anlagefläche 30 gedrückt, um einen möglichst kleinen Weg der Hohlnadel 5 in proximaler Richtung 21 zu ermöglichen. Eine Bewegung der Hohlnadel 5 in distaler Richtung 22 zum Herausziehen der Hohlnadel 5 ist dabei weiterhin möglich. Bei der Bewegung der Hohlnadel 5 in distaler Richtung 22 werden die Klemmflügel 27 gegen die Feder 29 gedrückt. Es entsteht keine Klemmwirkung durch die Sperrvorrichtung 2 gegen die Bewegung der Hohlnadel 5. Die Sperrvorrichtung 2 ist dabei innerhalb des Gehäuses 4 des Katheters 17 durch eine Rastnase 10 lösbar fixiert. Dabei ist die Kraft, die zum Lösen der Sperrvorrichtung 2 mit dem Käfig 3 von dem Gehäuse 4 nötig ist, größer als die Kraft, die durch das Herausziehen der Hohlnadel 5 aus dem Katheter 17 auf die Sperrvorrichtung 2 mit dem Käfig 3 wirkt, zumindest bis eine Quetschung 28 der Hohlnadel 5 an die Auflagefläche 11 des Käfigs 3 anstößt. Der Käfig 3 weist an ihrem distalen Ende einen Käfigdeckel 31 auf, der über ein Filmscharnier 32 mit dem Käfig 3 verbunden ist. Vorteilhaft nimmt der Feder-Klemmkonus der Sperrvorrichtung 2 je nach Status der Anwendung automatisch die richtige Einstellung/Schaltstellung ein.

## Patentansprüche

1. Verweilkanüle (1) mit einem flexiblen Katheter (17) und einer Hohlnadel (5), die mittels einer Überwurfmutter (33) mit einem Gehäuse (4) verbindbar ist, wobei der Katheter (17) innerhalb der Hohlnadel (5) längsverschiebbar ist und frei durch das Gehäuse (4) verläuft, **dadurch gekennzeichnet, dass** eine Verformung und/oder Beschädigung des Katheters (17) durch eine Sperrvorrichtung (2) während der Lagerung vermieden wird, wobei die Sperrvorrichtung (2) durch einen Feder-Klemmkonus mit einer Feder (29) und zwei Klemmflügeln (27, 27') ausgebildet ist, wobei die Feder (29) durch ein Führungselement (38) geführt wird und die Sperrung der proximalen und distalen Bewegung des Katheters (17) beim Transport und/oder der Lagerung durch die Sperrvorrichtung (2) mittels einer proximalen Transportsicherungsvorrichtung (34) und einer distalen Transportsicherung (35) über Stifte (36, 36'), die von beiden Seiten gegen die Feder (29) drücken, vermieden wird.

2. Verweilkanüle (1) nach Anspruch 1, **dadurch gekennzeichnet, dass** die Verweilkanüle (1) ein flexibler Katheter (17), insbesondere ein Periduralkatheter oder ein Seldingerdraht, ist.

3. Verweilkanüle (1) nach Anspruch 1 oder Anspruch 2, **dadurch gekennzeichnet, dass** die Schraubverbindung mittels der Überwurfmutter (33) zwischen der Hohlnadel (5) und dem Gehäuse (4) nicht mehr lösbar ist.

4. Verweilkanüle (1) nach einem der Ansprüche 1 bis 3, **dadurch gekennzeichnet, dass** der Feder-Klemmkonus der Sperrvorrichtung (2) je nach Status der Anwendung automatisch die richtige Einstellung/Schaltstellung einnimmt.

5. Verweilkanüle (1) gemäß einem der Ansprüche 1 bis 4, **dadurch gekennzeichnet, dass** die Sperrvorrichtung (2) ein Wiederzurückschieben des Katheters (17) in distaler Richtung (22) verhindert.

## Claims

1. Indwelling cannula (1) with a flexible catheter (17) and a hollow needle (5) which can be connected to a housing (4) by means of a union nut (33), the catheter (17) being longitudinally displaceable within the hollow needle (5) and extending freely through the housing (4), **characterised in that** deformation and/or damage to the catheter (17) is prevented by a locking device (2) during storage, the locking device (2) being formed by a spring-clamping cone with a spring (29) and two clamping wings (27, 27'), wherein the spring (29) is guided by a guide element (38) and the blocking of the proximal and distal movement of the catheter (17) during transport and/or storage is prevented by the blocking device (2) by means of a proximal transport securing device (34) and a distal transport securing means (35) via pins (36, 36') which press against the spring (29) from both sides.

2. indwelling cannula (1) according to claim 1, **characterised in that** the indwelling cannula (1) is a flexible catheter (17), in particular a peridural catheter or a Seldinger wire.

3. indwelling cannula (1) according to claim 1 or claim 2, **characterised in that** the screw connection by means of the union nut (33) between the hollow needle (5) and the housing (4) can no longer be loosened.

4. indwelling cannula (1) according to one of claims 1 to 3, **characterised in that** the spring-clamping cone of the locking device (2) automatically assumes the correct setting/switching position depending on the status of the application.

5. indwelling cannula (1) according to one of claims 1 to 4, **characterised in that** the locking device (2) prevents the catheter (17) from being pushed back in the distal direction (22).

## Revendications

1. Canule à demeure (1) avec un cathéter flexible (17) et une aiguille creuse (5) qui peut être reliée à un boîtier (4) au moyen d'un écrou-raccord (33), le cathéter (17) pouvant être déplacé longitudinalement à l'intérieur de l'aiguille creuse (5) et s'étendant librement à travers le boîtier (4), **caractérisée en ce qu'**une déformation et/ou un endommagement du cathéter (17) est évité par un dispositif de blocage (2) pendant le stockage, le dispositif de blocage (2) étant constitué par un cône de serrage à ressort avec un ressort (29) et deux ailes de serrage (27, 27'), le ressort (29) étant guidé par un élément de guidage (38), et le blocage du mouvement proximal et distal du cathéter (17) lors du transport et/ou du stockage étant évité par le dispositif de blocage (2) au moyen d'un dispositif de sécurité de transport proximal (34) et d'un dispositif de sécurité de transport distal (35) par l'intermédiaire de broches (36, 36') qui appuient des deux côtés contre le ressort (29).

2. Canule à demeure (1) selon la revendication 1, **caractérisée en ce que** la canule à demeure (1) est un cathéter flexible (17), en particulier un cathéter péridural ou un fil de Seldinger.

3. Canule à demeure (1) selon la revendication 1 ou la revendication 2, **caractérisée en ce que** la liaison par vis au moyen de l'écrou-raccord (33) entre l'aiguille creuse (5) et le boîtier (4) n'est plus détachable.

4. Canule à demeure (1) selon l'une des revendications 1 à 3, **caractérisée en ce que** le cône de serrage à ressort du dispositif de blocage (2) adopte automatiquement le réglage/la position de commutation correct(e) en fonction de l'état de l'utilisation.

5. Canule à demeure (1) selon l'une des revendications 1 à 4, **caractérisée en ce que** le dispositif de blocage (2) empêche le cathéter (17) d'être à nouveau repoussé dans la direction distale (22).
